# EUROPÄISCHE PATENTANMELDUNG

(11) **EP 1 273 305 A1**
(43) Veröffentlichungstag der Anmeldung: **08.01.2003**
(21) Anmeldenummer: 02013276.7
(22) Anmeldetag: 18.06.2002
(51) Int. Cl.: A61K 38/57, A61K 9/00, A61P 37/06, A61P 11/00

(54) **Arzneizubereitung zur Inhalation von Antithrombin bei entzündlichen Lungenerkrankungen und ARDS**

(30) Priorität: 06.07.2001 DE 10132307
(71) Anmelder: Aventis Behring GmbH, 35002 Marburg (DE)
(72) Erfinder: Hoffmann, Johannes, Dr., 80689 M-nchen (DE); Wiedermann, Christian, Prof., 6020 Innsbruck (AT); Roehmisch, Jürgen, Dr., 35041 Marburg (DE)

(57) **Zusammenfassung**

Es werden Arzneizubereitungen zur Inhalation bei entzündlichen Lungenerkankungen und akutem Lungenversagen (=ARDS) beschrieben, durch die Antithrombin III topisch appliziert wird.

## Beschreibung

Gegenstand der Erfindung ist eine Arzneizubereitung zur Prophylaxe und Therapie von entzündlichen Lungenerkrankungen und ARDS.

ARDS (=adult respiratory distress syndrome) ist eine Erkrankung, die auch als Schocklunge, akutes Lungenversagen, akute respiratorische Insuffizienz durch diffuse Schädigung der alveokapillären Membran bekannt ist. Trotz neuer vielversprechender Therapiestrategien ist sie immer noch durch eine sehr hohe Mortalität gekennzeichnet. Bei dem ARDS kommt es zu einer plötzlichen und progressiven Verschlechterung der Lungenfunktion, welche durch eine erhöhte Permeabilität der Lungen, assoziiert mit einer Hypoxämie bei herabgesetzter Lungencompliance und diffusen, beidseitigen pulmonalen Infiltraten in der Röntgen-Thorax-Aufnahme charakterisiert ist. Das ARDS kann als eine Folge einer direkten Schädigung des Lungenparenchyms oder indirekt in Assoziation mit einer Reihe von akuten systemischen Prozessen auftreten (1). Die Zerstörung der Lungenpermeabilität repräsentiert einen wichtigen Teil der akuten Lungenschädigung, wobei sich sowohl die chemische Zusammensetzung als auch die funktionelle Aktivität des Lungensurfactants bei Patienten mit ARDS verändert (2). Diese Symptome treten in ähnlicher Form auch bei anderen entzündlichen Lungenerkrankungen auf.

In der deutschen Patentanmeldung 44 34 629 sind bereits Zusammensetzungen zur Behandlung von IRDS und ARDS angegeben, die mindestens ein Glucocorticoid und ein Lungensurfactant enthalten. Die Behandlungsdauer und die von diesen Syndromen verursachte Mortalität kann durch derartige Arzneimittel gesenkt werden. Aus der internationalen Patentanmeldung WO 89/01341 sind bereits Verfahren und Arzneimittel zur Behandlung von Lungenmembranerkrankungen wie ARDS bekannt, in denen therapeutisch wirksame Mengen von γ-Interferon und/oder Tumor-Nekrosis-Faktor (TNF) entweder allein oder in Kombination mit Corticosteroiden eingesetzt werden. Hierdurch wird die Bildung von Lungensurfactants angeregt. Aus der internationalen Patentanmeldung WO 91/04054 ist der Einsatz von Antikörpern gegen TNF-α (anti-TNF) für Arzneimittel zur Behandlung oder Prophylaxe von ARDS bekannt.

Obwohl durch die Entwicklung derartiger neuer Therapiestrategien Fortschritte in der Behandlung von entzündlichen Lungenerkrankungen und des ARDS gemacht wurden, ist das ARDS noch immer mit einer hohen Mortalität verbunden, wobei die bisherigen Therapien weitgehend nur supportiv bleiben. Als kausale Ursache bietet sich zur Zeit lediglich die Möglichkeit der Behandlung mit Antibiotika zur Sanierung der Sepsisquelle an, ohne dadurch jedoch einen direkten Einfluss auf den inflammatorischen Prozess ausüben zu können. Eine mögliche Erklärung für die progressive Entwicklung einer Multiorgandysfunktion im Anschluss an ein akutes Lungenversagen könnte sein, dass es durch die maschinelle Beatmung zu einer Verstärkung der Entzündungsantwort in der Lunge kommt und dass dadurch inflammatorische Mediatorkaskaden aktiviert werden, welche den Gewebeschaden verstärken (3). So können beim ARDS abhängig vom Grad der Entzündung und der Phase des ARDS hohe lokale Konzentrationen von Mediatoren (Interleukin-6, Interleukin-1, Tumor-Nikrose-Faktor (TNF-α) nachgewiesen werden. Den lokal freigesetzten inflammatorischen Zytokinen scheint hierbei eine Schlüsselrolle bei der Verstärkung des Lungenschadens zuzukommen.

In großen Sepsisstudien der letzten 10 Jahre wurden spezifische und unspezifische, antiinflammatorische Substanzen wie Antikörper gegen Endotoxine und gegen Zytokine (anti-TNF, anti-Interleukin-6, Interleukin-1RA) sowie Modulatoren der Gerinnung (Antithrombin III, Protein C), getestet. Mit dieser systemischen Therapie wurde jedoch keine entscheidende Verbesserung der Organversagensparameter oder der Gesamtmortalität erreicht, wobei hohe Konzentrationen der antiinflammatorischen Substanzen systemisch verabreicht werden mussten, um therapeutische Spiegel in den Zielorgangen, zum Beispiel der Lunge, zu erreichen. So kam es nur unter der langdauernden systemischen Therapie mit Antithrombin zu einer Verbesserung der pulmonalen Funktion bei Patienten mit schwerer Sepsis, welche durch den Horowitz-Koeffizienten (pa O₂/FlO₂) charakterisiert wurde. Hierbei mussten sehr hohe Tagesdosen von Antithrombin systemisch verabreicht werden, zum Beispiel 6.000 I.E./Tag/Patient (4).

Es stellte sich deshalb die Aufgabe, nach effektiveren Behandlungsmöglichkeiten von entzündlichen Lungenerkrankungen und des ARDS zu suchen, die eine Verringerung der bisher erforderlichen Arzneimittelsdosis und damit auch eine Vermeidung systemischer Nebenwirkungen ermöglichen sollten. Es wurde nun gefunden, dass sich diese Möglichkeit durch die topische Applikation von Antithrombin III über eine Inhalationstherapie eröffnet.

Gegenstand der Erfindung ist deshalb eine Arzneizubereitung zur Inhalation gegen entzündliche Lungenerkrankungen und akutes Lungenversagen (=ARDS), die Antithrombin III enthält.

Die erfindungsgemäße Arzneizubereitung wird entweder in Pulverform zu inhalativen Verabreichung oder in flüssiger Form zur intratrachealen oder intrabronchialen Verabreichung zur Verfügung gestellt. Das Antithrombin III liegt in diesen Arzneizubereitungen in gelöster oder pulveriger Form vor und kann als Dosieraerosol, als Trockenaerosol oder als Nasalspray angewendet werden. Derartige Zubereitungen können neben dem Antithrombin III Verdünnungsmittel, Lösungsmittel oder auch Trägerstoffe enthalten, die die topische Applikation von Antithrombin III erleichtern.

Vorteilhaft sind auch Arzneizubereitungen, die das Antithrombin III zusammen mit einem Lungensurfactant und/oder mit einem Antiphlogistikum oder einem Glucocorticoid ausgewählt aus der Gruppe Betamethason, Methylprednisolon und/oder Dexamethason enthalten. Bei den Lungensurfactants handelt es sich vorzugsweise um ein hochgereinigtes, natürliches Surfactant aus homogenisierten Schweinelungen oder Rinderlungen sowie um Phospholipide. Flüssige Lungensurfactant-Zubereitungen werden zweckmäßigerweise vor oder nach der Zugabe des Glucocorticosteroids lyophilisiert und anschließend mikronisiert. Erfindungsgemäße Zusammensetzungen können bis zu etwa 10 Gewichtsprozent Glucocorticosteroide, bis zu 40% Lungensurfactants und bis zu 50% Antithrombin III enthalten. Besonders gute therapeutische Erfolge konnten mit Arzneizubereitungen erzielt werden, die Antithrombin III zusammen mit aktivierten Protein C, dem TFPI (=tissue factor pathway inhibitor) oder dem PAF-AH (=platelet activating factor Acylhydrolase) enthalten. Auch eine Kombination mit anderen Proteaseinhibitoren, die bspw. Elastase inhibieren, wie das Antitrypsin, SLPI (=secretory leukocyte protease inhibitor), Elafin oder entsprechenden synthetischen Inhibitoren ist vorteilhaft.

Die erfindungsgemäßen Zubereitungen werden 3 bis 4 mal täglich über 2 bis 4 Tage inhalativ, intratracheal oder intrabronchial verabreicht.

Durch die erfindungsgemäße Arzneizubereitung können in der Lunge wesentlich höhere Wirkspiegel an Antithrombin lokal erreicht werden, als durch jede andere Applikationsform. Während allen bisherigen Untersuchungen gemeinsam war, dass zur Erreichung von antiinflammatorischen Effekten, welche am ehesten über eine Interaktion mit Glycosaminglycanen an der Endothelzelloberfläche vermittelt werden, sehr hohe systemische Konzentrationen von Antithrombin III notwendig waren, um die antiinflammatorische Wirkung zu belegen (5), reichen bei der erfindungsgemäßen topischen Anwendung wesentlich geringere Wirkstoffkonzentrationen aus.
In klinischen Untersuchungen kann der Wert einer lokalen Vernebelung von vasodilatatorisch wirksamen oder antiinflammatorischen Substanzen belegt werden, wobei sich zum Beispiel durch die Gabe von NO kurzfristig eine Verbesserung des Gasaustausches erreichen ließ. Allerdings war durch die inhalative NO-Gabe keine Verbesserung der Lethalität des Lungenversagens erreichbar. Aufgrund der angenommenen Wirkmechanismen von Antithrombin III (lokale Prostazyklinfreisetzung und Herabregulation von antiinflammatorischen Zytokinen) ist anzunehmen, dass Antithrombin lokal appliziert antiinflammatorische Effekte vermittelt und eventuell sogar die Effektivität beider Maßnahmen verstärkt.

## Patentansprüche

1. Arzneizubereitung zur Inhalation gegen entzündliche Lungenerkrankungen und akutes Lungenversagen (=ARDS), **dadurch gekennzeichnet, dass** sie Antithrombin III enthält.

2. Arzneizubereitung nach Anspruch 1, **dadurch gekennzeichnet, dass** das Antithrombin III in gelöster oder in pulveriger Form vorliegt und als Dosieraerosol, als Trockenaerosol oder als Nasalspray angewendet werden kann.

3. Arzneizubereitung nach den Ansprüchen 1 und 2, **dadurch gekennzeichnet, dass** sie Antithrombin III zusammen mit Verdünnungsmitteln, Lösungsmitteln und/oder Trägerstoffen enthält.

4. Arzneizubereitung nach den Ansprüchen 1 bis 3, **dadurch gekennzeichnet, dass** sie das Antithrombin III zusammen mit einem Antiphlogistikum enthält.

5. Arzneizubereitung nach den Ansprüchen 1 bis 4, **dadurch gekennzeichnet, dass** sie das Antithrombin III zusammen mit aktiviertem Protein C und/oder dem TFPI (=tissue factor pathway inhibitor) und/oder Inhibitoren der Elastase und/oder dem PAF-AH (=platelet activating factor Acylhydrolase) enthält.

6. Arzneizubereitung nach Anspruch 5, **dadurch gekennzeichnet, dass** sie als Elastase-Inhibitor Antitrypsin, SLPI (=secretory leukocyte protease inhibitor), Elafin oder entsprechende synthetische Inhibtoren enthält.

7. Arzneizubereitung nach den Ansprüchen 1 bis 6, **dadurch gekennzeichnet, dass** sie das Antithrombin III zusammen mit Lungensurfactants und/oder mit einem Glucocorticoid ausgewählt aus der Gruppe Betamethason, Methylprednisolon und/oder Dexamethason enthält.

8. Verwendung von Antithrombin III zur Herstellung eines Inhalates oder eines Aerosoles nach den Ansprüchen 1 bis 7 zur Prophylaxe und Therapie bei akutem Lungenversagen.

9. Verwendung von Antithrombin III zur Prophylaxe und/oder Therapie durch topische Applikation bei entzündlichen Lungenerkrankungen und akutem Lungenversagen.
